(19) Europäisches Patentamt
European Patent Office
Office européen des brevets

(11) Publication number: 0 104 236
B1

(12) # EUROPEAN PATENT SPECIFICATION

(45) Date of publication of patent specification: 08.10.86

(51) Int. Cl.$^4$: C 07 F 1/02, B 01 J 31/12

(21) Application number: 83901455.2

(22) Date of filing: 10.03.83

(86) International application number: PCT/US83/00314

(87) International publication number: WO 83/03251 29.09.83 Gazette 83/23

(54) HYDROCARBON SOLVENT SOLUTIONS OF COMPLEXES OF (a) n-BUTYLLITHIUM, (b) SEC-BUTYLLITHIUM, AND (c) ISOPROPYLLITHIUM, WITH ETHYLLITHIUM.

(30) Priority: 15.03.82 US 357843
06.07.82 US 395272
06.07.82 US 395271
09.07.82 US 396815

(43) Date of publication of application: 04.04.84 Bulletin 84/14

(45) Publication of the grant of the patent: 08.10.86 Bulletin 86/41

(84) Designated Contracting States: BE CH DE FR GB LI NL

(56) References cited:
US-A-3 155 736
US-A-3 438 420
US-A-3 452 111
US-A-3 452 112

Weiner et al., JACS 85, 485-486, 1973

(73) Proprietor: LITHIUM CORPORATION OF AMERICA
449 North Cox Road
Gastonia NC 28053 (US)

(72) Inventor: MORRISON, Robert C.
1946 Elmwood Drive
Gastonia, NC 28052 (US)

(74) Representative: Berendt, Thomas, Dr.rer.nat. Dipl.-Chem. et al
Patentanwälte Dr.rer.nat. Dipl.-Chem. Th. Berendt Dr.Ing. Hans Leyh Innere Wiener Strasse 20/III
D-8000 München 80 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 104 236 B1

**Description**

Technical Field

The present invention deals with complexes of (a) n-butyllithium with ethyllithium, (b) sec-butyllithium with ethyllithium, and (c) isopropyllithium with ethyllithium and the method of preparation thereof.

Background Prior Art

It has heretofore been known, as disclosed in *J. American Chemical Society,* Vol. 84, pp. 485—6 (Weiner and West, 1973), in the article entitled "Complex Formation Between Ethyllithium and t-Butyllithium", to produce complexes of ethyllithium and t-butyllithium by dissolving ethyllithium and t-butyllithium in benzene. This article points out that ethyllithium alone is sparingly soluble in cold benzene (generally speaking, ethyllithium has a solubility of 0.2N in n-hexane and somewhat higher in cyclohexane at room temperature) but its solubility is greatly enhanced when the benzene solution contains t-butyllithium; and that the solution of the ethyllithium and the t-butyllithium forms a complex in which both the ethyl and t-butyl groups are bonded to lithium. The complexes are stated to be believed to be electron-deficient aggregates of the type $(EtLi)_n(t\text{-}BuLi)_{m-n}$, where m is a small number such as 4 or 6.

The said article further points out that, when benzene from a benzene solution of ethyllithium and t-butyllithium is evaporated, a low melting white solid residue is obtained which is highly soluble in pentane, unlike ethyllithium which is virtually insoluble in pentane; that the ratio of ethyl to t-butyl groups in the resublimed complex is nearly the same as in the original benzene solution; and that 1:1 ratios, as well as 1.8:1 ratios, of the ethyllithium to the t-butyllithium produced, on distillation, distilled products with ethyl:t-butyl ratios of 1.1:1 and 1.7:1, respectively, m.p. 68—72°C and 56—59°C. Benzene solutions specifically containing 8 wt.% each of ethyllithium and t-butyllithium are disclosed in said article.

Finally, the aforesaid article states that is was not known whether the formation of mixed organolithium compounds will occur generally, but that preliminary results indicate that t-butyllithium enhances the solubility of phenyllithium in benzene, but only to a limited extent. No uses or utilities are disclosed or suggested for the complexes of the ethyllithium and t-butyllithium or the benzene or pentane solutions thereof; or, for that matter, of the very generally disclosed phenyllithium solutions in benzene to which t-butyllithium was added.

As is pointed out in the article by Weiner, Vogel and West entitled "The Physical Properties and Structure of t-Butyllithium", *Inorganic Chemistry,* Volume 1, No. 3, August, 1962, pure t-butyllithium is a colorless crystalline solid, readily soluble in hydrocarbon solvents. Solutions of pure t-butyllithium in refluxing n-heptane turn brown and deposit a precipitate after about an hour, indicating that noticeable decomposition occurs during this time. A comparison is referred to with ethyllithium in relation to the degree of association, and it is stated that the average degree of association of t-butyllithium is very nearly four over a considerable range of concentrations in solutions in benzene and n-hexane, whereas the average degree of association in benzene solutions for ethyllithium is about six.

It has also been known to the art to prepare complexes (a) of ethyllithium with polyisoprenyllithium, (b) of n-butyllithium with polyisoprenyllithium, (c) of sec-butyllithium and polyisoprenyllithium, and, generally, of corresponding complexes where polystyryllithium was used in place of polyisoprenyllithium, in liquid hydrocarbon solvents such as benzene and n-hexane, Macromolecules, Vol. 3, No. 3, May, June, 1970, pp 333—337, article entitled "The Cross-Association of Polyisoprenyllithium with Ethyllithium", Morton, Patt and Fetters. These complexes have been prepared in connection with studies dealing with association phenomena in organolithium polymerizations in hydrocarbon solvents based upon the possibility of cross-association between the propagating polymerlithium species and any residual alkyllithium initiator since this could lead to complex kinetics in both the initiation and propagation reactions. Such studies were based upon concentration solution viscosity measurements. The authors reach the conclusion that the following association equilibrium can be written for this system:

$$(RM_2Li)_2 + (EtLi)_6 \rightleftharpoons 2RM_2Li \cdot (EtLi)_3$$

and that in the polymerization reaction preferential cross-association occurs.

Furthermore, it has been known, as disclosed in U.S. Patent No. 3,452,111, patented June 24, 1969 on an application filed December 9, 1966, which patent is owned by the Lithium Corporation of America, that the stability against decomposition of sec-butyllithium and other heat labile secondary alkyllithiums, notably those containiing from 4 to 8 carbon atoms in the alkyl radicals, generally in the form of solutions thereof in inert organic solvents, can be substantially increased by the addition thereto of variable proportions, specifically, of isopropyllithium and/or n-butyllithium. This patent does not deal in any way with ethyllithium, makes no reference whatever to the enhancement of the solubility of ethyllithium in inert organic solvent solutions thereof by the addition thereto of sec-butyllithium (or, for that matter, by the addition thereto of n-butyllithium or of isopropyllithium), or to the enhancement of the stability of sec-butyllithium or n-butyllithium or isopropyllithium by the addition thereto or the admixture therewith of ethyllithium, and said patent never contemplated the discoveries to which the present invention is directed.

It has also heretofore long been known and disclosed in numerous patents in connection with the preparation of homopolymers as well as copolymers of butadienes such as 1,3-butadiene; isoprene; vinyl-

substituted hydrocarbons; vinyl halides; vinylidene halides; esters of acrylic acid; esters of homologs of acrylic acid, and numerous other polymerizable monomers; as well as in the preparation of telomers, to utilize, as catalysts or initiators in such polymerization and telomerization reactions, metallic lithium; lithium hydrocarbons; alkyllithiums corresponding to the formula $R(Li)_x$ where R is a saturated or unsaturated hydrocarbon radical selected from aliphatic, cycloaliphatic, aralkyl, alkaryl and aromatic radicals, and x is an integer from 1 to 4, inclusive, and wherein the R group has a valence equal to the integer x and preferably contains from 1 to 20 carbon atoms. Examples thereof are methyllithium, ethyllithium, isopropyllithium, n-butyllithium, amyllithium, hexyllithium, t-octyllithium, cyclohexyllithium, s-butyllithium, t-butyllithium, and t-amyllithium; phenyllithium, tolyllithiums, xylyllithiums, alpha- and beta-naphthyllithiums, allyllithium, methallyllithium; hydrocarbon polylithium compounds such as methylene dilithium, ethylene dilithium, trimethylene dilithium, octadecamethylene dilithium, and 1,2-dilithium propane; polylithium aryl, aralkyl and alkaryl compounds such as 1,4-dilithium benzene, 1,5-dilithium naphthalene, 1,2-dilithium-1,3-diphenyl propane; tri- and higher lithium hydrocarbons such as 1,3,5-trilithium pentane and 1,3,5-trilithium benzene.

The foregoing organolithium compounds are disclosed in, among other patents, U.S. Patent Nos. 2,975,160; 3,065,218; 3,094,512; 3,231,635; 3,294,768; 3,297,793; 3,301,840; 3,317,918; 3,324,191; 3,332,856; 3,427,364; 3,449,306; 3,464,961; 3,465,065; 3,498,960; 3,513,056; 3,554,911; 3,558,575; 3,607,846; 3,652,516; 3,692,874; 3,742,077; 3,752,501; 3,760,025; 3,787,377; 3,840,616; 4,057,601; 4,076,914; and 4,237,245; Canadian Patent No. 750,006; and Australian Patent No. 262,782. The general statement is made in some of these patents, after listing many of the aforesaid organolithium compounds, including unsaturated organolithium compounds and aryl, aralkyl and alkaryl lithium compounds such as allyllithium, methallyllithium, phenyllithium, tolyllithiums, xylyllithiums and naphthyllithiums, that mixtures of such organolithium compounds can be used, but no particular examples of any such mixtures are given. Illustrative of such patents are U.S. Patent Nos. 3,506,631 and 3,632,563; and British Patent Nos. 817,693; 817,695; and 994,726. None of these patents provides any concept or teachings which would lead one versed in the art to the present invention and the important benefits which result therefrom.

With due regard to the foregoing disclosures in the above-enumerated patents concerning the uses, as catalysts or initiators, of any of large numbers of different organolithium compounds in polymerization and other reactions, or, as noted above, very generally stated mixtures of such organolithium compounds, it will be seen from said patents that, in practically all of them, in the actual disclosed working examples, with very minor exceptions, n-butyllithium is the catalyst or initiator of choice. This is because it has been found to be, with very limited exceptions, the most desirable organolithium compound from the overall standpoints of its effectiveness utility-wise for the intended reactions, its relative ease of handling and manufacture.

## Detailed Description

In connection with considerable investigatory work, it has been found that the behavior and properties of mixed alkyllithiums in hydrocarbon solvents, for instance, aliphatic or cycloaliphatic liquid hydrocarbon solvents, is unpredictable. Thus, in the case of methyllithium, for instance, whereas the complexes of n-butyllithium, or of sec-butyllithium, or of isopropyllithium, with ethyllithium of the present invention can highly effectively be prepared by the methods described below in the illustrative general procedure and working Examples of this specification, such procedures and methods are ineffective or essentially or practically inoperative for the preparation of complexes or t-butyllithium and methyllithium; or complexes of n-butyllithium, or sec-butyllithium, or isopropyllithium and methyllithium, nor by the mixing of independently-prepared t-butyllithium or independently-prepared n-butyllithium, or sec-butyllithium, or isopropyllithium, with independently-prepared methyllithium in liquid hydrocarbon solvents illustrative of which are n-hexane or cyclohexane. For convenience, n-butyllithium will sometimes hereafter be referred to as NBL; sec-butyllithium as SBL; isopropyllithium as IPL; ethyllithium as EL; the complexes of n-butyllithium with ethyllithium as NBEL; the complexes of sec-butyllithium with ethyllithium as SBEL; and the complexes of isopropyllithium with ethyllithium as IPEL.

It has been discovered, that (a) n-butyllithium, (b) sec-butyllithium, and (c) isopropyllithium each forms complexes with ethyllithium which have important advantages over n-butyllithium alone, or sec-butyllithium alone, or isopropyllithium alone, or ethyllithium alone, or over solutions of each of them alone in liquid hydrocarbon solvents, particularly aliphatic and/or cycloaliphatic hydrocarbon solvents, such as hexane or cyclohexane, as well as in their usefulness as catalysts or activators in polymerization and other reactions. It has been discovered that the addition of each of (a) n-butyllithium, or (b) sec-butyllithium, or (c) isopropyllithium, or mixtures of any two thereof, results in materially increasing the solubility of ethyllithium, when added to ethyllithium, in said solvents, exemplified particularly by hexane and cyclohexane. It has been further discovered that, for instance, the complexes which result from such mixtures of (a) n-butyllithium, (b) sec-butyllithium, or (c) isopropyllithium, or mixtures of two or more thereof, especially n-butyllithium, with ethyllithium, when dissolved in hydrocarbon solvents, notably n-hexane and/or cyclohexane, have exceptional and unexpected thermal stability, being very materially more thermally stable, for instance, than n-butyllithium, depending on the mole ratios of the n-butyllithium, or sec-butyllithium, or isopropyllithium, to the ethyllithium, and also depending on the concentrations of the complexes of the n-butyllithium, or sec-butyllithium, or isopropyllithium, with ethyllithium and also being,

to some extent, influenced by the particular hydrocarbon solvents in which said novel complexes are dissolved. Such thermal stability tests have been carried out at a temperature of 40°C and for time periods in excess of 100 days, for instance, of 111 and 119 days and longer periods. In the case of complexes of equimolar proportions, specifically, of sec-butyllithium and ethyllithium, and of 25 mole % sec-butyllithium and 75 mole % ethyllithium in dilute solutions in n-hexane or cyclohexane, such showed much greater thermal stability than sec-butyllithium alone.

Considering, first, the complexes of n-butyllithium and ethyllithium, it has been discovered that the stabilities of said complexes are very pronouncedly greater, at various temperatures, illustratively at 40°C, when such complexes are in the form of dilute solutions in liquid hydrocarbon solvents, illustratively, hexane or cyclohexane or mixtures thereof. Such dilute solutions may vary in their content of said complexes but, generally speaking, it is advantageous to market them in the form of solutions containing from 12 to 25 wt. %, or from 12 to 40 wt % of said complexes, particularly 15 to 25 wt. %. Thus, by way of illustration, dilute solutions of complexes of equimolar proportions of n-butyllithium and ethyllithium, and of complexes of 25 mole % n-butyllithium and 75 mole % ethyllithium in n-hexane demonstrated exceptional thermal stability, losing no C—Li activity after 4 months at 40°C. For the purposes of the present invention, dilute solution is considered as containing less than 40 wt. % of the complexes, and particularly advantageous, from a stability standpoint, being those containing from 12 or 15 to 25 wt. % of said complexes. Indeed, solutions containing 15 to 25 wt. % of equimolar proportions of n-butyllithium and ethyllithium, in hexane or cyclohexane, appear to be thermally stable indefinitely in sealed containers up to 40°C.

In the same vein, concentrated solutions of the complexes of the present invention may be considered as containing at least 40 wt. % of the complexes, and more commonly those containing from 65 to 95 weight % of the complexes, as high as 99 wt. % as noted below, depending upon the solubility of the particular complexes in given hydrocarbon solvents, while still maintaining substantial clarity of the solutions of said complexes. The solubility of the complexes is dependent also upon the mole percentages of the n-butyllithium, and/or sec-butyllithium, and/or isopropyllithium, with ethyllithium, of the particular complex and, as indicated above, upon the particular hydrocarbon solvent used. Thus, simply by way of illustration, where a complex of equimolar proportions by weight of the n-butyllithium and ethyllithium is initially prepared containing, say, 15 or 25 or under 40 wt. % of said complex dissolved in hexane or cyclohexane, such can be concentrated by evaporating off hexane to such an extent that the resulting concentrate contains at least 74 wt. % of said complex in the form of a clear solution.

In liquid hydrocarbon solutions containing increasing proportions of the complexes of the present invention, notably highly concentrated solutions of said complexes, the extent of the enhancement of the thermal stability of the complexes of the present invention, in comparison with the thermal stability of similarly concentrated solutions of n-butyllithium, is substantially reduced. Thus, tests of thermal stability of concentrated solutions, in hexane, of complexes of equimolar proportions of n-butyllithium and ethyllithium (63.6 wt. % RLi — 8.83 wt. % Li) showed that said solutions were just slightly more stable than concentrated solutions, in hexane, of n-butyllithium alone (87.4 wt. % RLi — 9.47 wt. % Li), in which case each sample contained similar amounts of lithium. For example, at 40°C, the concentrated solution of the complex lost 0.17% RLi/day, whereas the concentrated solution of the n-butyllithium alone lost 0.22% RLi/day. See, for instance, Table I. At still higher level of lithium content, an equimolar complex of n-butyllithium and ethyllithium (92.8 wt. % RLi — 12.88 wt. % Li) lost 0.26% RLi/day.

As indicated above, thermal stability tests were also carried out on liquid hydrocarbon solutions of different concentrations of n-butyllithium alone, at the illustrative temperature of 40°C. Furthermore, although, as previously stated, ethyllithium is very sparingly soluble in such liquid hydrocarbons as hexane and cyclohexane, thermal stability tests were conducted on a comparatively dilute, yet saturated, solution (0.4 N) of ethyllithium in cyclohexane, as noted below. So far as is known, prior to the present invention, no thermal stability data have been reported in the previous literature in regard to ethyllithium. A knowledge of its thermal stability properties is important because the complexes of the present invention generally, and especially advantageously, contain at least 50 mole % of ethyllithium. Prior to the present invention, ethyllithium, while per se being well known to the art, had essentially no commercial value because, apart from other considerations, of its very low solubility in liquid hydrocarbon solvents, as well as a relatively minimal knowledge concerning the scope of other alkyllithiums substantially to enhance its solubility in liquid hydrocarbon solvents, and a lack of knowledge by the prior art of the effect on the thermal stability of the complexes which might be formed between ethyllithium and other particular alkyllithiums.

Additional data in regard to thermal stability are reflected by the tests shown by the following Tables.

TABLE I

Comparison of Thermal Stability of Concentrated Solutions in Hexane of n-Butyllithium alone and of a Complex of Equimolar Proportions of n-Butyllithium and Ethyllithium at 40°C

| | Alkyllithium | Active RLi (wt.%) | Active Li (wt.%) | Total % Loss of Activity÷ | Days | Av % Loss/ Day |
|---|---|---|---|---|---|---|
| I | n-butyllithium | 87.4 | 9.47 | 19.6 | 89 | 0.22 |
| II | Equimolar Complex of n-butyllithium and ethyllithium | 63.6 | 8.83 | 14.3 | 85 | 0.17 |
| III | Equimolar Complex of n-butyllithium and ethyllithium | 92.8 | 12.88 | 12.5 | 47 | 0.26 |

The above data indicate that the complex in II is slightly more thermally stable than the n-butyllithium alone (I) if compared on the same lithium content basis. However, the thermal stability of the complex in the solution containing appreciably more active lithium (36%) (III) is slightly less than that of the n-butyllithium alone (I).

TABLE II

Thermal Stability of Concentrated n-Butyllithium in Hexane at 40°C Storage

| Days | Total Base (wt.%) | $V_2O_5$ (wt.%) | Loss of Active RLi(%) |
|---|---|---|---|
| 0(1) | 89.7 | 87.4 | 0 |
| 16 | 89.0 | 83.2 | 4.8 |
| 65(2) | 88.1 | 74.2 | 15.1 |
| 89(2) | 87.0 | 70.3 | 19.6 |

(1) Sample hazy
(2) Sample turbid

TABLE III

Thermal Stability of Concentrated Equimolar Complex of n-Butyllithium and Ethyllithium (I) at 40°C Storage

| Days | Total Base (wt.%) | $V_2O_5$ (wt.%) | Loss of Active RLi (%) |
|---|---|---|---|
| 0(2) | 68.0 | 63.6 | 0 |
| 16 | 66.3 | 60.7 | 4.6 |
| 65 | 72.3 | 64.3 | 0 |
| 85(3) | 61.9 | 54.5 | 14.3 |

(1) Initial concentration of complex = 63.6 wt %.
(2) Sample clear.
(3) Sample turbid.

TABLE IV
Thermal Stability of Concentrated Equimolar Complex of n-Butyllithium and Ethyllithium (1) at 40°C Storage

| Days | Total Base (wt.%) | $V_2O_5$ (wt.%) | Loss of Active RLi(%) |
|---|---|---|---|
| 0(2) | 94.5 | 92.8 | 0 |
| 11 | 95.5 | 92.5 | 0.3 |
| 39 | 95.5 | 83.9 | 9.6 |
| 47(3) | 93.1 | 81.2 | 12.5 |

(1) Initial concentration of NBEL 50:50 = 92.8 wt.%.
(2) Sample clear.
(3) Sample turbid.

TABLE V
Thermal Stability of Ethyllithium in Cyclohexane at 40°C

| Days | Total Base (N) | $V_2O_5$ (N) | Loss of Active RLi (%) |
|---|---|---|---|
| 0(1) | 0.44 | 0.41 | 0 |
| 22 | 0.41 | 0.40 | 2.4 |
| 64 | 0.44 | 0.41 | 0 |

(1) Sample clear.

It has been also found that complexes of equimolar amounts of n-butyllithium and ethyllithium in various hydrocarbon solvents as, for example, n-hexane or cyclohexane, can be concentrated by careful evaporation to produce clear concentrates of said complexes up to 90 wt. % or even higher with no precipitation. Preparation of such n-butyllithium and ethyllithium complexes, in the form of clear solutions in n-hexane, containing higher mole percentages of ethyllithium indicated that 1 n-butyllithium is needed to solubilize 5 ethyllithium. The concentration of these solutions at room temperature were ~ 1.0N. When ethyllithium is also prepared directly from ethyl chloride and a lithium dispersion, the resultant ethyllithium crystals can be dissolved and complexed with n-butyllithium in the same ratio (n-butyllithium/5 ethyllithium) as described above.

The relative proportions of the n-butyllithium and the ethyllithium are variable within reasonable limits, that is in a mole ratio of 1:9 n-butyllithium to 9:1 ethyllithium while maintaining solubility in the liquid hydrocarbon solvent. Thus, for example, equimolar amounts of n-butyllithium and ethyllithium are fully soluble in n-hexane. Relative mole proportions or 25 n-butyllithium and 75 ethyllithium are soluble to an extent to produce in the range of 15 to 25 wt. % in n-hexane. Relative mole proportions of 17 n-butyllithium and 83 ethyllithium are soluble to an extent to produce in the range of 5 to 10 wt. % solutions in n-hexane. In general, it is especially advantageous to utilize mixtures of n-butyllithium and ethyllithium, in solutions of the aforesaid hydrocarbon solvents, in which the mole ratio of the n-butyllithium to the ethyllithium is 50—50, or in the mole ratio of 35 n-butyllithium to 65 ethyllithium. Equimolar proportions of n-butyllithium and ethyllithium can produce clear solutions containing as high as 93 wt. % of such complexes at room temperature in n-hexane if one desires such highly-concentrated solutions. In cyclohexane, clear solutions can be obtained in wt. concentrations of the same complexes as high as 95 wt. % at room temperature. Complexes in which the mole ratio of n-butyllithium to ethyllithium is 36:64 can produce clear solutions having concentrations as high as 72% at room temperature. Complexes in which the mole ratio of the n-butyllithium to ethyllithium is 25:75 can produce clear solutions having concentrations as high as 23 wt. % in n-hexane and as high as 25 wt. % in cyclohexane. Complexes containing as low as 10 mole % n-butyllithium, balance 90 mole % ethyllithium, in some of said liquid hydrocarbon solvents can be obtained at a concentration of 4 wt. %, whereas ethyllithium can be obtained in 1 wt. % solution only.

In the case of the complexes of equimolar proportions of sec-butyllithium and ethyllithium, and of 25 mole % sec-butyllithium and 75 mole % ethyllithium in dilute solutions in n-hexane or cyclohexane, the

loss of activity was very greatly less than that of sec-butyllithium in the same solvents, in certain instances, said complexes showing a thermal stability of the order of 7 times that of sec-butyllithium, thus reducing substantially the refrigeration conditions for shipment and storage time considerations of hydrocarbon solvent solutions of said complexes versus those in relation to hydrocarbon solvent solutions of sec-butyllithium alone.

Further, in the case of the complexes of sec-butyllithium and ethyllithium, it has been discovered that the stability of said complexes is very pronouncedly greater, at various temperatures, illustrative at 40°C, when such complexes are in the form of dilute solutions in liquid hydrocarbon solvents, illustratively, hexane or cyclohexane or mixtures thereof. Such dilute solutions may vary in their content of said complexes but, generally speaking, it is advantageous to market them in the form of solutions containing from 12 to 25 wt. % of said complexes. Thus, by way of illustration, dilute solutions of complexes of equimolar proportions of sec-butyllithium and ethyllithium, and of complexes of 25 mole % sec-butyllithium and 75 mole % ethyllithium in n-hexane demonstrated exceptional thermal stability, losing less C—Li activity at 40°C than such solutions of the thermally less stable sec-butyllithium as such. Thus, for example, in the case of n-hexane solutions of complexes of equimolar proportions of sec-butyllithium and ethyllithium or complexes of 25 mole % of sec-butyllithium and 75 mole % of ethyllithium, the loss of RLi activity occurs at the rate of 0.2% per day at 40°C, which is 25% of the loss per day of the RLi in n-hexane solutions of sec-butyllithium of the same wt. % of lithium. Complexes in liquid hydrocarbon solvent solutions, as in hexane or cyclohexane, have also been made in accordance with the present invention containing 17 mole % of sec-butyllithium and 83 mole % of ethyllithium (0.81N).

In this same vein, more concentrated solutions of the sec-butyllithium/ethyllithium complexes, up to saturation, which may contain up to 61 to 99 weight % of said complexes, can be prepared, depending upon the solubility of the particular complexes, in given hydrocarbon solvents, while still maintaining substantial clarity of the solutions of said complexes. The solubility of such complexes is dependent also upon the mole percentages of the sec-butyllithium and ethyllithium of the particular complex and, as indicated above, upon the particular hydrocarbon solvent used. Thus, simply by way of illustration, where a complex of equimolar proportions by weight of the sec-butyllithium and ethyllithium is initially prepared containing, say, 15 or 25 wt. % of said complex dissolved in hexane or cyclohexane, such can be concentrated by evaporating off hexane to such an extent that the resulting concentrate contains up to 99 wt. % of said complex in the form of a clear solution; whereas a 25:75 mole ratio of sec-butyllithium and ethyllithium can be concentrated to a 50 wt. % solution. At elevated temperatures, the concentrated solutions of such complexes of the present invention are less stable than the dilute solutions of said complexes, and when stored in glass bottles at room temperature both the concentrated and dilute solutions of the complexes appear to be more stable than sec-butyllithium based on observation of the appearance of the sample contents with time.

As indicated above, thermal stability tests were also carried out on liquid hydrocarbon solutions of different concentrations of sec-butyllithium alone, at the illustrative temperature of 40°C.

Additional data in regard to thermal stability of SBEL complexes are reflected by the tests shown by the following Tables.

TABLE VI
Thermal Stability of SBEL 50:50 in Cyclohexane

| | 50 Mole % SBL:50 Mole % EL 40° Storage | | |
|---|---|---|---|
| Days | Total Base (wt.%) | $V_2O_5$ (wt.%) | Loss of Active RLi (%) |
| 0[(1)] | 17.0 | 14.6 | 0 |
| 44 | 16.1 | 12.9 | 11.6 |

(1) Sample clear.

TABLE VII
Thermal Stability of SBEL 25:75 in Cyclohexane

25 Mole % SBL:75 Mole % EL
40° Storage

| Days | Total Base (wt.%) | $V_2O_5$ (wt.%) | Loss of Active RLi (%) |
|---|---|---|---|
| 0(1) | 15.1 | 13.4 | 0 |
| 35 | 14.5 | 12.7 | 5.2 |

(1) Sample clear.

TABLE VIII
Thermal Stability of SBL in Cyclohexane
40° Storage

| Days | Total Base (wt.%) | $V_2O_5$ (wt.%) | Loss of Active RLi (%) |
|---|---|---|---|
| 0(1) | 12.5 | 12.2 | 0 |
| 9(2) | 12.0 | 10.7 | 12.3 |
| 30 | 11.1 | 9.04 | 25.9 |

(1) Sample clear.
(2) Sample turbid containing a precipitate.

From the foregoing Tables VI, VII and VIII, it will be seen that a 12.2 wt. % solution of SBL in cyclohexane suffered at 25.9% loss of active RLi after 30 days at 40°C, whereas a 14.6 wt. % solution of SBEL (50:50) suffered a loss of 11.6.% of active RLi after 44 days, while a 13.4 wt. % solution of SBEL (25:75) suffered a loss of 5.2% of active RLi after 35 days. In other tests, a 50:50 SBEL solution in cyclohexane, at 40°C for 42 days, showed an RLi loss per day of 0.17%; and a 25:75 SBEL solution in cyclohexane, at 40°C for 37 days, showed an RLi loss per day of 0.20%.

As has been noted above, complexes of equimolar amounts of sec-butyllithium and ethyllithium, and complexes of 25 mole % of sec-butyllithium and 75 mole % of ethyllithium, in various hydrocarbon solvents as, for example, n-hexane or cyclohexane, can be concentrated by careful evaporation to produce clear concentrates of the respective complexes to 99 wt. % and 50 wt. % with no precipitation. Preparation of such sec-butyllithium and ethyllithium complexes, in the form of clear solutions in n-hexane or other hydrocarbon solvents containing higher mole percentages of ethyllithium indicated at 1 sec-butyllithium is needed to solubilize 5 ethyllithium. The concentrations of these solutions were ~ 1.0 N. When ethyllithium is also prepared directly from ethyl chloride and a lithium dispersion, the resultant ethyllithium crystals can be dissolved and complexed with sec-butyllithium in the same ratio (sec-butyllithium/5 ethyllithium) as described above.

The relative proportions of the sec-butyllithium to ethyllithium are variable within reasonable limits, that is in a molar ratio of 17:83 to 95:5 while maintaining solubility in the liquid hydrocarbon solvent. Thus, for example, equimolar amounts of sec-butyllithium and ethyllithium are fully soluble in normally liquid hydrocarbon solvents such as n-pentane, n-hexane and cyclohexane. Relative mole proportions of 25 sec-butyllithium and 75 ethyllithium are soluble to an extent to produce in the range of 40 to 50 wt. % in such solvents. Relative mole proportions of 17 sec-butyllithium and 83 ethyllithium are soluble to an extent to produce in the range of 5 to 10 wt. % solutions in such solvents. In general, it is especially advantageous to utilize mixtures of sec-butyllithium and ethyllithium, in solutions of the aforesaid hydrocarbon solvents, in which the mole ratio of the sec-butyllithium to the ethyllithium is 50—50, or in the mole range of 35 sec-butyllithium to 65 ethyllithium. Equimolar proportions of sec-butyllithium and ethyllithium can produce clear solutions containing as high as 99 wt. % or slightly higher of such complexes at room temperature in n-hexane or cyclohexane or other hydrocarbon solvents. As the mole ratio of sec-butyllithium to ethyllithium decreases, the solubility limit of the corresponding complex also decreases.

In the case of the complexes of 25 mole % isopropyllithium and 75 mole % ethyllithium in dilute solutions in n-hexane or cyclohexane, tests show a thermal stability about double that of isopropyllithium. In the case of complexes of equimolar proportions of isopropyllithium and ethyllithium, the thermal stability was of the same order as that of isopropyllithium alone.

In general, the stability of the complexes of isopropyllithium and ethyllithium tend to be greater, at various temperatures, illustratively at 40°C, when such complexes are in the form of dilute solutions in liquid hydrocarbon solvents, illustratively, hexane or cyclohexane or mixtures thereof. Such dilute solutions may vary in their content of said complexes but, generally speaking, it is advantageous to market them in the form of solutions containing from 12 to 40 wt. % of said complexes, particularly 15 to 25 wt. %. Thus, by way of illustration, dilute solutions of complexes of 25 mole % isopropyllithium and 75 mole % ethyllithium, in solution in cyclohexane, stored at 40°C over a period of 37 days, showed an RLi loss per day of 0.16%; whereas a complex comprising equimolar proportions of isopropyllithium and ethyllithium showed an RLi loss per day, stored at 40°C over a period of 42 days, of 0.25%.

In this same vein, concentrated solutions, considered as containing at least 40 wt. % of the IPEL complexes, and more commonly those containing from 60 to 65 weight % of said complexes, depending upon the solubility of the particular complexes in given hydrocarbon solvents, while still maintaining substantial clarity of the solutions of said complexes, tend to be less stable than dilute solutions. The solubility of said complexes is generally dependent upon the mole percentages of the isopropyllithium and ethyllithium of the particular complex and, as indicated above, upon the particular hydrocarbon solvent used. Where a complex of equimolar proportions by weight of the isopropyllithium and ethyllithium is initially prepared containing, say, 15 or 25 or under 40 wt. % of said complex dissolved in hexane or cyclohexane, such can be concentrated by evaporating off hexane to such an extent that the resulting concentrate contains 73 wt. %, more or less, of said complex in the form of a clear solution.

As indicated above, thermal stability tests were also carried out in liquid hydrocarbon solutions of different concentrations of isopropyllithium alone, at the illustrative temperature of 40°C.

The following Tables IX and X disclose tests run in regard to the thermal stability of complexes of equimolar proportions of isopropyllithium and ethyllithium (denoted in Table IX as IPEL 50:50), and complexes of 25 mole % isopropyllithium and 75 mole % ethyllithium (denoted in Table X as IPEL 25:75) each in wt. % proportions of ~ 5% in cyclohexane. The thermal stability testing method employed in the tests in Tables IX and X was the prior art standard testing procedure (hereinafter described) where a single cylinder was filled with the complex, the cylinder being opened at periodic intervals and samples removed for testing on given days during the test period.

TABLE IX

Thermal Stability of IPEL 50:50 in Cyclohexane

| | 40°C Storage | | |
|---|---|---|---|
| Days | Total Base (N) | $V_2O_5$ (N) | Loss of Active RLi (%) |
| 0(1) | 0.93 | 0.85 | 0 |
| 7 | 0.93 | 0.85 | 0 |
| 21 | 0.91 | 0.79 | 7.0 |
| 28 | 0.89 | 0.76 | 10.5 |
| 35(2) | 0.89 | 0.74 | 12.9 |
| 42 | 0.89 | 0.76 | 10.5 |

(1) Sample clear containing no precipitate.
(2) Sample turbid containing a precipitate.

TABLE X
Thermal Stability of IPEL 25:75 in Cyclohexane

40°C Storage

| Days | Total Base (N) | $V_2O_5$ (N) | Loss of Active RLi (%) |
|---|---|---|---|
| 0(1) | 0.93 | 0.82 | 0 |
| 9 | 0.94 | 0.83 | 0 |
| 16 | 0.92 | 0.79 | 3.6 |
| 23 | 0.91 | 0.78 | 4.8 |
| 30(2) | 0.88 | 0.78 | 4.8 |
| 37 | 0.88 | 0.77 | 6.1 |
| 51 | 0.88 | 0.74 | 9.7 |
| 65(2) | 0.88 | 0.70 | 14.6 |

(1) Sample clear containing no precipitate.
(2) Sample hazy containing a small amount of precipitate.

With respect to IPEL complexes of equimolar amounts of isopropyllithium and ethyllithium in various hydrocarbon solvents as, for example, n-hexane or cyclohexane, it has been also found that such can be concentrated by careful evaporation to produce clear concentrates of said complexes (containing 73 wt. % in n-hexane and 67 wt. % in cyclohexane) with no precipitation. Preparation of such IPEL complexes, in the form of clear solutions in said solvents, containing higher mole percentages of ethyllithium indicated that 1 mol isopropyllithium is needed to solubilize 5 mol ethyllithium. The concentrations of these solutions were 1.0 N. When ethyllithium is also prepared directly from ethyl chloride and a lithium dispersion, the resultant ethyllithium crystals can be dissolved and complexed with isopropyllithium in the same ratio (isopropyllithium/5 ethyllithium) as described above.

The relative proportions of the isopropyllithium and the ethyllithium are variable. The ratio of the isopropyllithium to the ethyllithium is, on a mole basis, from 1:9 to 9:1. The extent of the solubility limits of said complexes, while maintaining clarity of the solution, will depend upon the particular isopropyllithium-ethyllithium complex, the particular hydrocarbon solvent, and the particular temperature of the solvent. Thus, for example, equimolar amounts of isopropyllithium and ethyllithium are highly soluble in n-hexane and in cyclohexane. Relative mole proportions of 25% isopropyllithium and 75% ethyllithium are soluble to an extent to produce a somewhat lower wt. % in n-hexane and in cyclohexane. In general, it is advantageous to utilise mixtures of isopropyllithium and ethyllithium, in solutions of the aforesaid hydrocarbon solvents, in which the mole ratio of the isopropyllithium to the ethyllithium is 50:50, or 35:65, or 25:75. Equimolar proportions of isopropyllithium and ethyllithium can produce clear solutions containing as high as about 73 wt. % of such complex at room temperature in n-hexane if one desires such highly-concentrated solutions; while, in cyclohexane, clear solutions can be obtained in wt. concentrations of the same complex as high as 67 wt. % at room temperature.

As indicated above, the NBEL, SBEL and IPEL complexes of the present invention, particularly in solution in aliphatic and/or cycloaliphatic hydrocarbon solvents, are highly useful in various reactions where n-butyllithium solutions, or sec-butyllithium solutions or isopropyllithium solutions, in such solvents have been used, such as initiator/catalysts for stereospecific polymerization of conjugated dienes, such as butadiene and isoprene, and for copolymerization of dienes with vinyl aromatic compounds such as styrene, such as are disclosed in the patents listed above, and wherein, in certain of such polymerization reactions, the polymerization reactions proceed more rapidly than when ethyllithium alone or n-butyllithium is used as a catalyst or initiator; as well as for use as cocatalysts for low pressure (Ziegler) polymerization of α-olefins; as well as in other reactions such as metalation reactions; and in the preparation of magnesium alkyls such as are disclosed in U.S. Patent Nos. 3,646,231 and 3,755,478. In the production of magnesium alkyls, they can be reacted with various alkylmagnesium chlorides, $R_2Mg{\cdot}MgCl_2$, which results from the reaction of n-alkylhalides and magnesium metal in liquid hydrocarbon solvents. Processes such as are disclosed in U.S. Patent Nos. 4,069,267 and 4,127,507 can be carried out to produce

dialkylmagnesiums utilizing the complexes of the present invention, particularly useful for such purposes being those complexes which comprise equimolar proportions of NBEL, SBEL of IPEL in solution in a liquid hydrocarbon solvent.

In the preparation of the NBEL, SBEL and IPEL complexes, dissolved in aliphatic and/or cycloaliphatic hydrocarbon solvents, there is the further significant advantage over the preparation on n-butyllithium, sec-butyllithium or isopropyllithium alone, as the case may be, by reason of the fact that they possess an overall lower molecular weight than n-butyllithium, sec-butyllithium or isopropyllithium alone, as the case may be, which results in increasing the capacity of a preparation installation. In addition, production costs are substantially reduced since ethyl halides are materially lower in cost on a molar basis than the costs of the other alkyl halides.

While n-hexane and cyclohexane represent the liquid hydrocarbon, particularly the aliphatic and cycloaliphatic hydrocarbon, solvents of choice in which the NBEL, SBEL and IPEL complexes of the present invention are used, and are prepared for use, various other inert aliphatic or aromatic hydrocarbon solvents or mixtures thereof which can be used, although generally less preferred, which will usually contain from 5 to 12 carbon atoms are n-pentane, 2,4-dimethylhexane, octane, isooctane, n-decane, n-dodecane, methylcyclohexane, benzene, toluene, n-propyl benzene, isopropylbenzene, and xylenes. Mixtures of such liquid hydrocarbons can be employed as, for example, mixtures of n-hexane and cyclohexane in various proportions to each other such as 50—50 mixtures, 75—25 mixtures, and 60—40 mixtures. Generally speaking, however, it is more desirable to use a single liquid hydrocarbon solvent since no particular advantage is generally achieved by using mixtures thereof.

In the preparation of the NBEL, SBEL and IPEL complexes in accordance with the present invention, it has been found to be especially satisfactory to prepare them directly by reacting (a) a premixture of n-butyl chloride or bromide and ethyl chloride or bromide; or (b) a premixture of sec-butyl chloride or bromide and ethyl chloride or bromide; or (c) a premixture of isopropyl chloride or bromide and ethyl chloride or bromide; most desirably in the form of a relatively homogeneous premixture in each case, with a stirred dispersion or slurry of finely divided lithium metal in a liquid hydrocarbon, particularly of aliphatic or cycloaliphatic character, such as n-hexane or cyclohexane, in which the complexes produced are soluble, said reaction being advantageously carried out at a temperature in the range of 20 to 35°C, usually between 28 and 32°C. It is particularly desirable that the reaction be initiated by initially adding to the stirred lithium metal dispersion or slurry a small amount, usually not more than what is equivalent to 2 wt. % of the total of the particular premixed alkyl halides involved, of an initiator, after which the said premixture is added gradually, under conditions of relatively vigorous stirring, until the reaction for producing said complexes is completed. The stirring is then discontinued and the unused or excess lithium metal is allowed to rise to the top of the reaction mass from where it is then drawn off. The "muds" which form during the reaction, consisting primarily or essentially of lithium chloride (or lithium bromide if n-butyl bromide or sec-butyl bromide or isopropyl bromide, and ethyl bromide, are utilized), are separated from the liquid of the reacted mass, desirably by filtering, and said muds are washed one or more times with a given volume of the liquid hydrocarbon solvent, to recover from said muds the complexes adhering thereto or carried thereby. The washings may be added to the prior filtrate comprising the clear solution of the complexes in the liquid hydrocarbon solvent.

Alternatively, in the carrying out of the above-described method, instead of allowing the excess lithium metal to rise to the surface and be drawn off after the completion of the reaction to produce the complexes and then separately separating the muds, as by filtration, the entire mass including the lithium metal and the muds is filtered off and separated from the filtrate comprising the solution of the complexes in the hydrocarbon solvent, and the separated solids are washed with the hydrocarbon solvent to recover the complexes carried by the separated solids.

As noted above, a homogeneous premixture of n-butyl chloride (or sec-butyl chloride or isopropyl chloride), in the selected predetermined proportions, is added to the lithium metal slurry in the carrying out of the reaction to produce the desired complexes of the n-butyllithium (or sec-butyllithium or isopropyllithium, as the case may be) and ethyllithium. Instead of so proceeding, one may gradually feed simultaneously, separately, into the lithium metal slurry, the n-butyl chloride (or the sec-butyl chloride or the isopropyl chloride, as the case may be) and the ethyl chloride, at rates so as to correspond to the desired selected predetermined proportions to produce the particular aforesaid complexes. This approach, however, is distinctly less desirable and not preferred for a variety of reasons including handling difficulties.

No novelty is claimed per se in the preparation of the lithium metal dispersion proper in the hydrocarbon solvent. This is conveniently done by vigorous stirring or admixing finely divided lithium metal in an inert liquid medium, most desirably a mineral oil, illustratively a mineral oil such as that sold under the trade name designation PRIMOL 155®, or an admixture thereof with petrolatum, for instance, in proportions of 25 to 35 volume % of the lithium metal, balance mineral oil or mineral oil admixed with some petrolatum. Mineral oil, alone, as the dispersing agent for the finely divided lithium metal is preferred. The lithium metal advantageously is admixed with or is made so as to contain a small percentage of sodium metal, e.g., 0.5 to 2%, preferably 0.7% to 1.7%, which serves to enhance the formation of the aforesaid complexes of the present invention. To some extent, small proportions of other metals can be used in place of or in conjunction with sodium as, for example, potassium, rubidium, cesium

or calcium, but sodium metal is especially useful. No novelty, however, is claimed per se broadly in using lithium metal admixed with a small proportion of sodium metal (and/or said other metals) in reactions with an alkyl halide to produce an alkyllithium since this is, in itself, known to the art. When reference is made to lithium metal in lithium metal dispersions, it will be understood to mean lithium metal containing or admixed with a small proportion, e.g., of the order of at least 0.5% and up to 2% more or less of sodium metal (and/or generally equivalent metal). The foregoing generally known procedural aspects are shown in various of the above-mentioned patents as well as in U.S. Patent No. 3,452,112. All operations, to the extent reasonably feasible, are carried out under inert gas conditions or in an inert gas atmosphere, such as argon, since, as is well known in the art, air, oxygen and moisture are detrimental to reactions of the type disclosed in the present invention and the presence thereof is to be avoided to the maximum extent reasonably possible.

In the practise of the method of the present invention for the preparation of the novel NBEL, SBEL and IPEL complexes, high yields of said complexes are commonly obtained, generally of the order of 88 or 90 to 95%. Thus, by utilizing the foregoing novel procedure, clear solutions of said complexes are directly formed in the particularly desired aliphatic or cycloaliphatic liquid hydrocarbon solvents, whereas, if the n-butyl chloride or bromide (or sec-butyl chloride or bromide, or isopropyl chloride or bromide) and the ethyl chlorde or bromide are similarly reacted separately in the manner otherwise described, results would occur which would cause severe handling and manipulative problems.

Furthermore, by carrying out the production of the aforesaid NBEL, SBEL and IPEL complexes in the novel and advantageous manner described above, the additional important advantage is achieved of rendering unnecessary the use of strong Lewis Bases, e.g., ethers. In fact, and of definite significance, the simultaneously formed soluble species n-butyllithium or sec-butyllithium or isopropyllithium apparently solubilizes the insoluble species ethyllithium as it forms, thereby keeping the lithium metal surface clean and allows the reaction readily to proceed to completion, without complications. The preparation of the NBEL, SBEL and IPEL complexes in accordance with the present invention has the still further advantage that, due to the lower molecular weight of said complexes, as compared to that of n-butyllithium (or sec-butyllithium or isopropyllithium), more C—Li equivalents/kg per batch is obtained which, as generally noted above, results in an increase of plant capacity per unit of time.

The following examples are illustrative of the preparation of the complexes of NBEL, SBEL and IPEL made in accordance with the present invention. An illustrative general procedure will first be described followed by specific examples.

In said illustrative procedure and in said examples, the following described apparatus or equipment set-up was employed. It will, of course, be understood that this is simply exemplary and those skilled in the art can readily evolve modified apparatus set-ups particularly in connection with scale-up operations for large or commercial plant production of the complexes. All temperatures recited are in °C; and all percentages referred to are in terms of mole percentages unless otherwise expressly stated.

Apparatus

250 ml or 500 ml round bottom reaction flask (3 neck)
Dry-ice condenser
250 ml jacketed dropping funnel ($CO_2$-hexane cooled)
Thermometer (0 to 100°C)
Mechanical stirrer
Cooling bath ($CO_2$-hexane)
250 ml glass filter funnel (medium porosity)
250 ml or 500 ml round bottom receiving flask (3 neck)

Note: All glassware was baked in an oven at 150°C for at least 12 hours prior to use. The apparatus was then set-up hot and cooled with an argon purge.

Materials Used (except as specifically otherwise stated in the examples) for the preparation of the NBEL complex

| | |
|---|---|
| 13.4 ml | Ethylchloride (etCl) (0.1875 moles) |
| 6.5 ml | n-Butylchloride (n-BuCl) (0.0625 moles) |
| 13.0 g | Lithium dispersion in petrolatum and Primol 155® (30 wt.%—0.56 g. atoms) |
| 200 ml | n-Hexane (solvent) |
| 80 ml | n-Hexane (dispersion wash) |
| 5 ml | n-Butyllithium (n-BuLi) in hexane (14.8 wt.%)—Li metal conditioner |

Materials Used (except as specifically otherwise stated in the examples) for the preparation of the SBEL complex

| | |
|---|---|
| 16.1 g | Ethylchloride (EtCl) 0.25 moles) |
| 23.1 g | sec-Butylchloride (sec-BuCl) (0.25 moles) |
| 26 g | Lithium dispersion in petrolatum and Primol 155® (30 wt.%—1.1 g. atoms) |
| 200 ml | Cyclohexane (solvent) |
| 140 ml | Cyclohexane (dispersion wash) |
| 5 ml | SBEL (50:50) in Cyclohexane—(0.004 moles)—Li metal conditioner |

Materials Used (except as specifically otherwise stated in the examples) for the preparation of the IPEL complex

| | |
|---|---|
| 16.1 g | Ethylchloride (EtCl) (0.25 moles) |
| 19.6 g | Isopropylchloride (IPCl) (0.25 moles |
| 26.0 g | Lithium dispersion in petrolatum and Primol 155® (30 wt.%—1.1 g. atoms) |
| 300 ml | Cyclohexane (solvent) |
| 100 ml | Cyclohexane (dispersion wash) |
| 5 ml | n-Butyllithium (n-BuLi) in n-hexane (1.1 N)—Li metal conditioner |

Illustrative General Procedure
For The Preparation of An NBEL Complex

Preparation of NBEL from Premixed n-BuCl (or Br) and EtCl (or Br)

NBEL is prepared by reacting a premixture of n-BuCl and EtCl and a dispersion of finely divided lithium metal in a liquid hydrocarbon such as n-hexane or cyclohexane at a temperature of 25° to 35°. The chemistry is reflected by the following:

$$CH_3—(CH_2)_2—CH_2Cl+CH_3—CH_2Cl+4Li \xrightarrow[H.C.]{25—35°C}$$

$$\underbrace{CH_3—(CH_2)_2—CH_2—Li \quad + \quad CH_3—CH_2—Li}_{NBEL} + 2LiCl$$

A premixture of N-BuCl and EtCl, in predetermined mole proportions in relation to each other, as described above, is added to a vigorously stirred dispersion of finely divided metallic lithium under an inert gas atmosphere. Prior to the addition of the premixed alkyl halides, to insure high yields of the NBEL, an excess of the lithium metal dispersion is used, for instance, of the order of 10% to 15%, said excess being based upon the excess stoichiometric amount of the lithium metal in relation to the premixture of the n-BuCl and EtCl. The lithium metal used in the dispersion contains, or is admixed with, as a part of the dispersion in a small amount, based on the weight of the lithium metal, of sodium metal, generally in the amount indicated above in the specification. Prior to the carrying out of the reaction to produce the NBEL, the lithium metal of the dispersion is conditioned or activated by stirring into the hydrocarbon slurry a small proportion of NBL or previously produced NBEL, which need not exceed 2 wt.% of the total charge of the premixture of the n-BuCl and EtCl, for a period of time which is variable but which may be of the order of about an hour or so. The exothermic alkyl chloride-metal reaction is initiated at once. On heat being noted, the premixture of the n-BuCl and EtCl is added gradually or dropwise at a rate necessary to keep the reaction mass temperature preferably at 30°. The reaction is highly exothermic and cooling is generally

necessary. After reaction and, desirably, a postreaction period of time, of the variable order of a half hour or more, the NBEL solution is separated from the muds (LiCl and excess Li) by filtration and the muds are washed with n-hexane. NBEL is analyzed by total base (LiOH) and vanadium pentoxide (active C-Li) assays. Recovered yields are determined by the following method:

$$\text{Volume (liter)} \times \text{N. } (V_2O_5) = \text{eq. C-Li}$$

$$\frac{\text{eq. C-Li (bound)}}{\text{moles halide used}} \times 100 = \text{\% Yield}$$

Essentially, the same illustrative general procedure is used for the preparation of the SBEL and IPEL complexes, except that sec-butyl chloride is used in place of n-butyl chloride for the preparation of the SBEL complexes; and except that isopropyl chloride is used in place of the n-butyl chloride for the preparation of the IPEL complexes. The chemistry for the SBEL and IPEL complexes is reflected by the following:

$$\text{Sec-BuCl} + \text{EtCl} + 4\text{ Li} \xrightarrow[\text{H.C.}]{30°} \underbrace{\text{Sec-BuLi} + \text{EtLi}}_{\text{SBEL}} + 2\text{ LiCl}$$

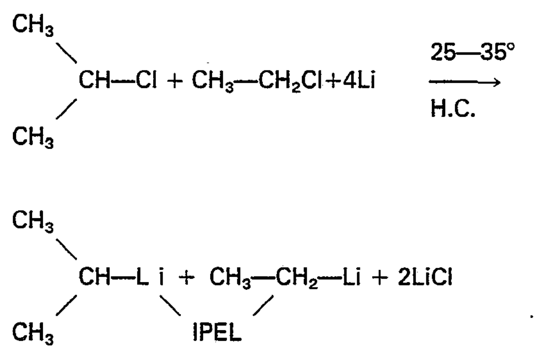


## Example 1

13 g of a finely divided lithium metal in petrolatum-Primol 155® and 80 ml dry n-hexane are charged with the assembled and argon purged apparatus. The resulting slurry is stirred for 15 minutes and stirring is then stopped. The lithium is allowed to rise to the top of the solution. The 80 ml of n-hexane wash containing the oil and petrolatum is then removed by syringe from the bottom of the flask. This is replaced by the reaction solvent (150 ml dry n-hexane). The jacketed dropping funnel is cooled with the dry-ice/n-hexane mixture. Then the 13.4 ml of gaseous EtCl is condensed into the funnel. Next, the 6.5 ml BuCl is added to the contents of the dropping funnel. The resulting mixture is shaken vigorously to insure complete mixing. The reaction is initiated by adding 1 ml of the mixed halides to the stirred lithium slurry. An immediate rise in temperature (3°) indicates spontaneous initiation. Addition of another 1 ml of the mixed halides solution brings the pot temperature to 30°. The dry-ice/hexane cooling bath is then set in place. The n-BuCl/EtCl premixture is added dropwise over a period of about 44 minutes. The reaction is very sensitive to the mixed halide addition but is controlled easily by the cooling bath ($\Delta$ = 10° to 15°). The resultant reaction mass is slowly stirred overnight. Filtration of the final product and a 50 ml hexane wash of the muds requires about 2 minutes. A volume of 235 ml of a clear, colorless solution of NBEL is recovered.

Analysis

| | |
|---|---|
| Total Base | = 1.03N |
| Active Alkyl ($V_2O_5$) | = 1.00 N |
| Yield | = 94% (recovered) |

The following additional examples, Table XI, show other runs made for the preparation of NBEL solutions in n-hexane using other mole ratios of n-BuCl/EtCl. In these examples, a refluxing hydrocarbon solvent mixture of n-butane (B.P.—0.5°) and n-hexane at 30° is the reaction medium. The excellent yields are due at least in part to efficient heat transfer caused by the vaporization of the n-butane at the site of

reaction, namely, the lithium-metal surface. Efficient removal of the intense heat of reaction all but eliminates the competing reaction of Wurtz coupling. In NBEL preparations, the presence of volatile ethyl chloride (B.P. 13.1°C) possibly may also be responsible for the same beneficial heat transfer. Although very little condensation is noted in the condenser, immediate fogging appears above the reaction slurry upon the addition of the mixed halides. The NBEL obtained from the above examples is crystal clear after filtration.

TABLE XI

Preparation of NBEL in n-Hexane

| Ex. No. | n-BuCl (Moles) | EtCl (Moles) | Butyl Ethyl (Ratio) | Lithium g (Atoms) | Metal % (Excess) | n-Hexane Ml | n-BuLi (1) (Moles) | Analysis Total Base (N) | Analysis Active Alkyl (N) | Recovered Yield (%) |
|---|---|---|---|---|---|---|---|---|---|---|
| 2 | 0.125 | 0.125 | 1:1 | 0.58 | 14 | 150 | 0.007 | 1.33 | 1.30 | 95.0 |
| 3 | 0.025 | 0.225 | 1:9 | 0.58 | 14 | 150 | 0.007 | 1.02 | 0.98 | 100.0 |
| 4 | 0.035 | 0.207 | 17:83 | 0.58 | 14 | 150 | 0.007 | 0.99 | N.A | 92.1 |

(1) Amount of n-butyllithium used to condition or activate the lithium dispersion.

In the following TABLE XII, illustrative examples are shown of the preparation of NBEL solutions in cyclohexane.

TABLE XII

Preparation of NBEL in Cyclohexane

| Ex. No. | n-BuCl (Moles) | EtCl (Moles) | n-Butyl-Ethyl (Mole Ratio) | Lithium Dispersion | | Reaction Solvent | | Metal Conditioner (1) | | Analysis | | Recovered Yield (%) |
|---|---|---|---|---|---|---|---|---|---|---|---|---|
| | | | | g (Atoms | % (Excess) | (Type) | (ml) | (Type) | (Moles) | Total Base (N) | Active Alkyl (N) | |
| 5 | 0.50 | 0.50 | 1:1 | 2.2 | 10 | Cyclo-hexane | 250 | n-BuLi | 0.008 | 1.79 | N.A. | 99.4 |
| 6 | 0.25 | 0.25 | 1:1 | 1.1 | 10 | Cyclo-hexane | 250 | n-BuLi | 0.008 | 0.96 | 0.93 | 98.0 |

(1) The amount of n-BuLi used to condition the lithium dispersion prior to reaction.

The runs of Examples 5 and 6 initiate well and are sensitive to the halide feed throughout the entire addition. Filtrations are rapid, yielding light yellow, clear solutions. The final product obtained from Example 6 is concentrated to 74.1 wt.% (Total Base) via vacuum on a Rinco Flask Evaporator. The active alkyl analysis by $V_2O_5$ is 71.5 wt.% (Active C-Li = 96.5%). The concentrate is a liquid. At −25°C, the viscosity increases but does not cause any crystallization. In terms of lithium content, 74.1 wt. % NBEL (1:1) contains more active lithium (Li = 10.4 wt.%) than does 90 wt.% conc. n-BuLi (Li = 9.8 wt.%). This is due to the lower molecular weight of NBEL (M. wt. = 50) *vs.* conc. n-BuLi (M. wt. = 64). In any event, NBEL (1:1), produced pursuant to my present invention, is producible in excellent yield in cyclohexane or hexane and can be concentrated to at least 94 wt.%.

Example 7

26 g of a finely divided lithium metal in petrolatum-Primol 155® and 140 ml dry cyclohexane are charged with the assembled and argon purged apparatus. The resulting slurry is stirred for 15 minutes and stirring is then stopped. The lithium is allowed to rise to the top of the solution. The 140 ml of cyclohexane wash containing the oil and petrolatum is then removed by syringe from the bottom of the flask. This is replaced by the reaction solvent (150 ml dry cyclohexane) and 5 ml of SBEL. The mixture is then stirred for 2 hours to insure activation of the lithium metal. The jacketed dropping funnel is cooled with the dry-ice n-hexane mixture. Then 16.1 g of gaseous EtCl is condensed into the addition funnel. Next, the 23.1 g sec-BuCl is added to the contents of the dropping funnel. The resulting chloride mixture is shaken vigorously to insure complete mixing. The reaction is initiated by adding ~ 1 ml of the mixed halides to the stirred lithium slurry. An immediate rise in temperature (3°) indicates spontaneous initiation. Addition of another 1 ml of the mixed halides solution brings the pot temperature to 30°. The dry-ice hexane cooling bath is then set in place. The sec-BuCl-EtCl premixture is added dropwise over a period of about 80 minutes. The reaction is very sensitive to the halide addition but is controlled easily by the cooling bath (Δ T = 10 to 15°C). The resultant reaction mass is slowly stirred overnight. Filtration of the final product requires about 2 minutes. A volume of 183 ml of a clear, colorless solution of SBEL is recovered. The muds are washed separately with 50 ml of cyclohexane. The wash contains 0.049 eq. of SBEL.

| Analysis | |
|---|---|
| Total Base | = 2.48N |
| Active Alkyl ($V_2O_5$) | = 2.47N |
| Yield | = 99.2% (recovered) |

The following additional Examples, Table XIII, show other runs made for the preparation of SBEL solutions in n-hexane and in cyclohexane using other molar ratios of sec-BuCl-EtCl.

TABLE XIII

Preparation of sec-Butyllithium-Ethyllithium (SBEL)

| Ex. No. | sec-BuCl (Moles) | EtCl (Moles) | sec-Butyl: Ethyl (Mole Ratio) | Lithium g (Atoms) | Metal % (Excess) | Reaction solvent (Type) | Reaction solvent (ml) | Metal Conditioner (1) (Type) | Metal Conditioner (1) (Moles) | Analysis Total Base (N) | Analysis Active Alkyl (N) | Recovered Yield (%) |
|---|---|---|---|---|---|---|---|---|---|---|---|---|
| 8 | 0.085 | 0.415 | 17:83 | 1.1 | 10 | Hexane | 150 | BEL (50:50) | 0.005 | 0.82(2) | N.A. | 90.3 |
| 9 | 0.25 | 0.25 | 1:1 | 1.1 | 10 | Cyclo-hexane | 150 | BEL (50:50) | 0.005 | 2.45 | 2.40 | 95.2 |
| 10 | 0.25 | 0.25 | 1:1 | 1.1 | 10 | Cyclo-hexane | 250 | n-BuLi | 0.006 | 0.77 | 0.75 | 87.8 |
| 11 | 0.50 | 1.50 | 1:3 | 4.4 | 10 | Hexane | 1300 | Conc. BuLi | 0.040 | 1.23 | 1.05 | 91.4 |

(1) The amount of RLi used to condition the lithium dispersion prior to reaction.
(2) This value represents the solubility limit of SBEL (17:83). The remaining insoluble SBEL left on the filter is solubilized with sec-BuLi in order to determine the recovered yield.

In Example 8, a halide mole composition of 17% sec-butyl chloride and 83% ethyl chloride was reacted, and filtration of the final product yielded an 0.82N solution of SBEL. This may be considered to be the solubility limit of an SBEL containing 5 moles ethyllithium and 1 mole sec-butyllithium. This Example 8 demonstrates that small amounts of sec-butyllithium can substantially enhance the solubility of the sparingly soluble ethyllithium.

In Example 9, a cyclohexane solution (2.4 N) of SBEL 50:50 was prepared from a 1:1 mole ratio of sec-butyl chloride and ethyl chloride, the recovered yield being 95.2%. The product showed no signs of deterioration (no precipitation) after 55 days of storage at 0°C. A similar preparation of SBEL (50:50) in cyclohexane resulted in an 87.8% recovered yield (Example 10). The SBEL from Example 10 was concentrated from 5.4 wt.% to 66.1 wt.% via vacuum on a Rinco Flask Evaporator. At −25°C, the viscosity of the concentrate increased to almost a glass but did not crystallize. It may be noted that a 66.1% SBEL (50:50) contains the same amount of active lithium (Li = 9.3 wt.%) as an 85.4 wt.% concentration of n-butyllithium (Li = 9.3 wt.%) due to the lower molecular weight of SBEL (M.wt. = 50) vs. n-butyllithium (M.wt. = 64). Also, samples of SBEL 50:50 were carefully concentrated to 99 wt.% and also remained clear.

Example 12

26 g of a finely divided lithium metal in petrolatum-Primol 155® and 100 ml dry cyclohexane are charged with the assembled and argon purged apparatus. The resulting slurry is stirred for 15 minutes and stirring is then stopped. The lithium is allowed to rise to the top of the solution. The 100 ml of cyclohexane wash containing the oil and petrolatum is then removed by syringe from the bottom of the flask. This is replaced by the reaction solvent (200 ml dry cyclohexane). The jacketed dropping funnel is cooled with the dry-ice n-hexane mixture. Then the 16.1 g of gaseous EtCl is condensed into the funnel. Next, the 19.6 g IPCl is added to the contents of the dropping funnel. The resulting mixture is shaken vigorously to insure complete mixing. The reaction is initiated by adding 1 ml of the mixed halides to the stirred lithium slurry. An immediate rise in temperature (3°) indicates spontaneous initiation. Addition of another 1 ml of the mixed halides solution brings the pot temperature to 33°. The dry-ice hexane cooling bath is then set in place. The IPCl-EtCl premixture is added dropwise over a period of about 98 minutes. The reaction is very sensitive to the chloride addition but is controlled easily by the cooling bath ($\Delta T = 20°$). The resultant reaction mass is slowly stirred overnight. Filtration of the final product and two 50 ml cyclohexane washes of the muds requires 3 minutes. A volume of 345 ml of a clear, colorless solution of IPEL is recovered.

| Analysis | |
|---|---|
| Total Base | = 1.39N |
| Active Alkyl ($V_2O_5$) | = 1.33N |
| Yield | = 95.9% (recovered) |

The following additional Examples, Table XIV, show other runs made for the preparation of IPEL solutions in n-hexane and in cyclohexane using other molar ratios of IPCl-EtCl. The IPEL obtained from the above Examples is crystal clear after filtration.

TABLE XIV

Preparation of Isopropyllithium—Ethyllithium (IPEL)

| Ex. No. | Isopropyl-Chloride (Moles) | EtCl (Moles) | Isopropyl-Ethyl (Mole Ratio) | Lithium g (Atoms) | Dispersion % (Excess) | Reaction Solvent (1) (Type) | Metal Conditioner (2) (ml) | Metal Conditioner (2) (Type) | Metal Conditioner (2) (Moles) | Analysis Total Base (N) | Analysis Active Alkyl (N) | Recovered Yield (%) |
|---|---|---|---|---|---|---|---|---|---|---|---|---|
| 13 | 0.25 | 0.25 | 1:1 | 1.1 | 10 | Cyclo-hexane | 200 | n-BuLi | 0.005 | 1.39 | 1.33 | 95.9 |
| 14 | 0.25 | 0.25 | 1:1 | 1.1 | 10 | Hexane | 200 | n-BuLi | 0.005 | 1.34 | 1.23 | 84.4 |
| 15 | 0.125 | 0.375 | 1:3 | 1.1 | 10 | Cyclo-hexane | 200 | n-BuLi | 0.005 | 1.51 | 1.46 | 99.9 |
| 16 | 0.125 | 0.375 | 1:3 | 1.1 | 10 | Hexane | 200 | conc. BuLi | 0.020 | 1.21 | 1.11 | 98 |
| 17 | 0.50 | 0.50 | 1:1 | 2.2 | 10 | Cyclo-hexane | 400 | Conc. | 0.010 | 1.56 | 1.46 | 100 |

(1) Does not include the volume of solvent used for washing the reaction muds. The wash generally is one-half the volume used for the reaction.
(2) The amount of RLi used to condition the lithium dispersion prior to reaction.

With respect to the above-discussed thermal stability of the NBEL, SBEL and IPEL hydrocarbon solutions, a brief explanation as to the manner in which the tests with respect thereto were carried out is believed to be in order. Because of the known readiness with which alkyllithiums degrade chemically due to their high rate of reaction with oxygen and water, with the resulting lowering C-Li activity, as well as causing other problems, the thermal stability tests reported herein of said solutions in liquid hydrocarbon solvents were carried out in a manner such as to minimize contact with $O_2$ and $H_2O$ and to obtain a distinctly more accurate picture of the true thermal stability properties of said NBEL, SBEL and IPEL solutions. Thermal decomposition of alkyllithiums proceeds by lithium hydride elimination:

$$RLi \xrightarrow{\Delta} Olefin+LiH$$

The matter of thermal stability of alkyllithiums is highly important because the rate of thermal degradation dictates the way alkyllithiums must be handled, stored and shipped.

Thermal stability of alkyllithium is commonly carried out by placing samples thereof in rolled steel cylinders (492 and 901 $cm^3$ volume). Prior to use, each cylinder is equipped with the appropriate fittings to insure a leak proof system and then is "pickled" or conditioned with the alkyllithium. The alkyllithium solution in the hydrocarbon solvent is then placed in the cylinder and analyzed for total base and active RLi ($V^2O^5$ method). The sample is then placed in a constant temperature bath at 40°C (±1°C). Periodically, the cylinder is removed from the bath, opened and reanalyzed for total base and active RLi. From these data, the thermal stability of the alkyllithium is calculated and plotted on graph paper (% loss active RLi vs. time). Since the cylinder is opened and sampled several times during the course of the test, the chance for atmospheric oxygen and $H_2O$ contamination is great. This can often result in erroneously high thermal stability data because the loss due to contamination is additive. In addition, lithium alkoxide formed by the reaction of $O_2$ with alkyllithium increases the rate of the thermal degradation of organolithiums. In the present case, all thermal stability procedure tests were carried out by a modification of the above-described method whereby the contamination problem described above was at least largely circumvented. This was done by using several cylinders of the NBEL, SBEL and IPEL hydrocarbon solutions to be tested. The tests were carried out as described above except that each cylinder was sampled only once. This procedure materially reduced the chance for atmospheric contamination, thus providing a more accurate thermal stability evaluation.

The following examples are illustrative of polymerization, telomerization and other reactions which are effectively carried out by the utilization of complexes of NBEL, SBEL and IPEL in liquid hydrocarbon solutions made in accordance with the present invention.

The hydrocarbon solutions of the NBEL, SBEL and IPEL complexes of the present invention can, in general, be used in place of soutions of n-butyllithium, sec-butyllithium and isopropyllithium in known polymerization and copolymerization reactions to produce liquid as well as solid polymers and copolymers, oligomers, triblock polymers, "Star" polymers, metalation and other reactions. The following examples are illustrative of such usage. All temperatures cited are in °C.

## Example A

Preparation of Polystyrene

To an oven-dried one pint sample bottle, purged with $N_2$, is added 150 ml of dry cyclohexane. The bottle is then capped with a septum. Using a syringe, 16.5 ml of freshly distilled sytrene (inhibitor free) is added to the cyclohexane. Next, the reaction vessel is placed in a constant temperature bath at 36.5°C and continuously mixed using a shaker. 0.001 mole of the solution of the complex made in accordance with Example 1 is then added via syringe, followed by sealing of the septum with parafilm. A pale yellow color appears in a few seconds after said addition. The color of the solution becomes orange in approximately 30 seconds. The color intensity gradually increases with time and becomes a deep red after 12 hours. The polymerization is terminated after 24 hours with 2 ml of degassed isopropanol. A one ml sample of the resulting colorless solution is concentrated using a gentle $N_2$ purge. A resulting colorless film of polystyrene is obtained.

## Example B

Preparation of Polystyrene

To an oven-dried one pint sample bottle purged with $N_2$, is added 150 ml of dry cyclohexane. The bottle is then capped with a septum. Using a syringe, 16.5 ml of freshly distilled styrene (inhibitor free) is added to the cyclohexane. Next, the reaction vessel is placed in a constant temperature bath at 36.5°C and continously mixed using a shaker. 0.001 mole of the solution of the complex made in accordance with Example 7 is then added via syringe, followed by sealing of the septum with parafilm. The color of the solution becomes orange in approximately 30 seconds. The color intensity gradually increases with time and becomes a deep red after 12 hours. The polymerization is terminated after 24 hours with 2 ml of degassed isopropanol. A one ml sample of the resulting colorless solution is concentrated using a gentle $N_2$ purge. A resulting colorless film of polystyrene is obtained which is then subjected to GPC analysis for determination of $M_n$, $M_w$, $M_z$, and corresponding molecular weight distributions (MWD). Based on the

calculated MWD's of the polystyrene obtained using the above procedure, the SBEL initiator produced a polydispersity of 1.2 to 1.3, as compared to that of n-butyllithium which was 1.5. As a check for the above procedure for the preparation of polystyrene, sec-butyllithium was also tested in the same manner and a MWD of 1.05 was obtained. This MWD for sec-butyllithium is in agreement with existing data for sec-butyllithium as an initator for use in the production of polystyrenes.

Example C

Preparation of Polystyrene

To an oven-dried one pint sample bottle (0,5 l) purged with $N_2$, is added 150 ml of dry cyclohexane. The bottle is then capped with a septum. Using a syringe, 16.5 ml of freshly distilled styrene (inhibitor free) is added to the cyclohexane. Next, the reaction vessel is placed in a constant temperature bath at 36.5°C and continuously mixed using a shaker. 0.001 mole of the solution of the complex made in accordance with Example 12 is then added via syringe, followed by sealing of the septum with parafilm. A pale yellow color appears in a few seconds after said addition. The color of the solution becomes orange in 30 seconds. The color intensity gradually increases with time and becomes a deep red after 12 hours. The polymerization is terminated after 24 hours with 2 ml of degassed isopropanol. A one ml sample of the resulting colorless solution is concentrated using a gentle $N_2$ purge. A resulting colorless film of polystyrene is obtained. The film of polystyrene is then subjected to GPC analysis for determination of $M_n$, $M_w$, $M_z$, and corresponding molecular weight distributions. Based on the calculated MWD's of the polystyrenes obtained using the above procedure, the IPEL initiators produced a polydispersity of 1.1 to 1.3 compared to that of n-butyllithium which was 1.5. As a check for the procedure above, isopropyllithium was also tested in the same manner. The MWD for isopropyllithium is in agreement with existing data for this initiator.

Example D

Preparation of Telomer

To 400 ml of toluene 1.3 g (0.0116 mole) of potassium t-butoxide and 2.9 ml of a concentrated (90%) solution in hexane of the complex made in accordance with the procedure of Example 1 are added under conditions of stirring and in the absence of oxygen and moisture. Then 1,3-butadiene is added as a gas at a flow rate of 3.5 liters/min. The temperature rises to about 60—65° and is maintained in that range throughout the reaction with external cooling. After 3 hours, the reaction is terminated by the addition of 2 ml of water. The telomeric product is a pale yellow mobile liquid.

Example E

Metal-Halogen Exchange

Under an argon atmosphere, 1 g of p-bromotoluene is dissolved in 50 ml tetrahydrofuran (freshly distilled from lithium aluminum hydride) and the solution is cooled to −78°C. One equivalent consisting of a complex of 50 mole % n-butyllithium or sec-butyllithium and 50 mole % ethyllithium in hexane is added over a 2 minute period with stirring. After 45 minutes, the reaction is quenched with 1 ml anhydrous methanol, followed by removal of the cooling bath. Proton NMR analysis of the resulting solution reveals that 99+% metal-halogen takes place. Also, evidence for the occurrence of alkylation is not detected.

Example F

Metal-Halogen Exchange

Under an argon atmosphere, 1 g of p-bromoanisole is dissolved in 50 ml of freshly distilled tetrahydrofuran and the solution is cooled to −78°C. One equivalent of the complex, which consisted of 50 mole % isopropyllithium and 50 mole % ethyllithium in hexane, is added over a 2 minute period with stirring. After 30 minutes, the reaction is quenched with 1 ml anhydrous methanol, followed by removal of the cooling bath. GC analysis of the resulting solution reveals that greater than 95% metal-halogen exchange had taken place. Also, any occurrence of alkylation could not be detected. High yields are also obtained when the above procedure is repeated using 3-bromothiophene and other arylbromides such as meta and ortho-bromoanilsole, and o-bromo-α,α.α-trifluorotoluene.

Example G

Metal-Proton Exchange

The following example relates to the preparation of 2,6-dimethoxybenzoic acid. A solution of 0.20 mole of SBEL (50:50) or (25:75) is added dropwise over a 30 minute period to a room temperature solution of 0.30 mole of 1,3-dimethoxybenzene. After 8 hours, the resulting mixture of 2,6-dimethoxyphenyllithium is slowly drained into a hexane-toluene slurry of dry ice in large excess. The resulting carboxylation is continued for 12 hours at −50°C, then gradually allowed to warm to room temperature. The tan colored mixture of lithium 2,6-dimethoxybenzoate is slowly quenched by pouring it into 400 ml of water. The resulting mixture is stirred with a magnetic stirrer for 30 minutes. The pale yellow aqueous layer is separated and decolorized with activated charcoal. The nearly colorless aqueous solution is acidified (pH ~ 1) with 6N HCl to cause precipitation of the product. The crystalline product is filtered and dried to give a 92% yield of 2,6-dimethoxybenzoic acid (m.p. 186—188°C uncorrected; lit. 188—190°C).

Example H

Preparation of DMBA from Dimethylresorcinol

To a room temperature solution of 0.24 moles DMR in 55 ml hexane is added dropwise over a 45 minute period a solution of 0.2 mole of the complex solution of Example 1. After stirring overnight, the resulting yellow to orange colored mixture of DMPL (dimethoxy phenyl lithium) is slowly drained into a slurry of 80 ml hexane, 70 ml toluene, and 600 g dry ice (large excess of $CO_2$). the carboxylation is continued at −50°C for 12 hours, then gradually allowed to warm to room temperature. The tan colored mixture of lithium 2,6-dimethoxybenzoate is slowly quenched by pouring into 400 ml of tap water. The resulting mixture is stirred with a magnetic stirrer for 30 minutes. The pale yellow aqueous layer is separated and decolorized with 1 g activated charcoal. The resulting filtered solution, which is a nearly colorless solution is acidified (PH 1) with 6N HCl to cause precipitation of DMBA. The crystalline product is filtered and dried. Then the melting point is determined followed by calculation of percent yield based on moles of the complex of Example 1 used. Analysis of the yield of 2,6-dimethoxyphenyllithium resulting from the metalation step is determined by proton NMR. Using this method, a sample of the mixture resulting from the reaction of complex metalating agent and 1,3-dimethoxybenzene is quenched with deuterium oxide. The proton NMR spectrum reveals greater than 95% ±2% deuterium incorporation.

The steps in the foregoing procedure for the production of DMBA according to Example H are shown below.

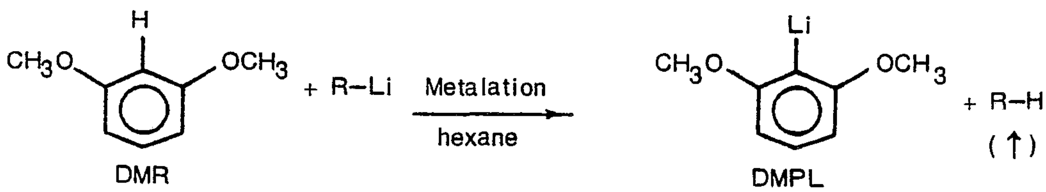


DMR DMPL

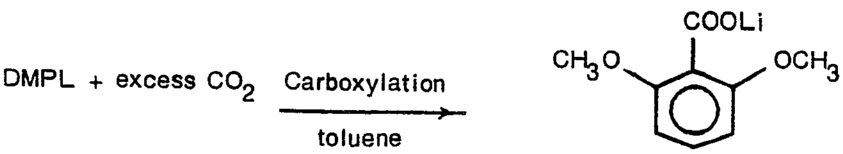


Lithium 2,6-dimethoxybenzoate

(I)

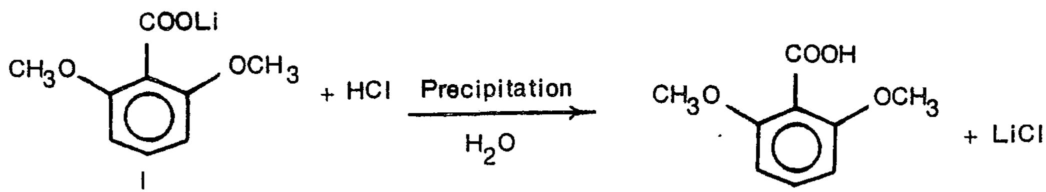


I DMBA

Some observations may be made with respect to the production of DMBA as set forth in Example H. While good yields of DMBA are obtained by known prior art procedures obtained with the use of hydrocarbon solutions of n-butyllithium in such known processes (~ 90%), said processes, so far as is known, require the utilization of a substantial excess, generally about 24%, in the metalation step. By the process of Example H, savings are effected not only through the use of the more economical complexes of n-butyllithium and ethyllithium in place of n-butyllithium, but it has also been found that the amount of excess DMR normally required in the aforesaid heretofore known plant production process (which excess DMR is expensive, is not recovered, but is apparently required to produce good yields of DMBA based on the limiting n-butyllithium), but it has also been found that the amount of excess DMR normally required in the aforesaid heretofore known plant production process can be substantially reduced, for instance, to the order of 5%, thus further appreciably reducing manufacturing costs since good yields of high quality DMBA are obtained comparable with those obtained using n-butyllithium. This is illustrated by the foregoing Example H.

**Claims**

1. A method of preparing liquid hydrocarbon solutions of complexes selected from (a) butyllithium, (b) sec-butyllithium and (c) isopropyllithium, together with ethyllithium, in which the ratio, on a mole basis, of the n-butyllithium to the ethyllithium is from 1:9 to 9:1; of the sec-butyllithium to the ethyllithium is from 17:83 to 95:5; of the isopropyllithium to the ethyllithium is from 1:9 to 9:1; which comprise the steps providing a stirred dispersion of finely divided lithium metal containing a small proportion of sodium metal in a liquid hydrocarbon selected from aliphatic and cycloaliphatic hydrocarbons with which the lithium metal is essentially unreactive, gradually adding simultaneously to said lithium metal dispersion, under conditions of vigorous agitation and in an inert gas atmosphere, (a') n-butyl chloride or bromide, (b') sec-butyl chloride or bromide, (c') isopropyl chloride or bromide, with ethyl chloride or bromide in predetermined relative proportions to provide the aforesaid solutions containing the complexes (a), (b) or (c) in their above-stated mole ratios, and continuing the reaction at a temperature in the range of 20° to 35°, under conditions of stirring, until said reaction is at least substantially completed, and filtering to produce a substantially clear solution of said complexes (a), (b) or (c) in said hydrocarbon solvent.

2. The method of Claim 1, in which the mole ratio of (a), (b) or (c) to ethyllithium in said complexes is substantially equimolar.

3. The method of Claim 1, in which the mole ratio of (a), (b) or (c) to ethyllithium is 35 to 65.

4. The method of Claim 1, in which the mole ratio of (a), (b) or (c) to ethyllithium is 25 to 75.

5. The method of Claim 1, in which the lithium content of said slurry is in excess of the stoichiometric amount thereof in relation to the amounts of (a'), (b') or (c'), as the case may be.

6. The method of Claim 1, in which the weight percentage of said (a), (b) or (c) complexes in said hydrocarbon solvent is such as to produce a dilute solution thereof.

7. The method of Claim 6, in which said hydrocarbon solvent is selected from aliphatic and/or cycloaliphatic hydrocarbon solvents.

8. The method of Claim 7, in which the mole ratio of the n-butyllithium to the ethyllithium in said complexes is equimolar.

9. The method of Claim 7, in which the mole ratio of the n-butyllithium to the ethyllithium is 25 to 75.

10. The method of Claim 1, in which the mole ratios of (a), (b) or (c) to the ethyllithium in said complexes are selected from (a) about equimolar, (b) 35 to 65, and (c) 25 to 75, and in which the weight percentages of said complexes in said hydrocarbon solvent are such as to produce a dilute solution thereof.

11. A composition comprising a clear solution in a liquid hydrocarbon solvent of a complex selected from (a) n-butyllithium, (b) sec-butyllithium and (c) isopropyllithium, together with ethyllithium, in which the ratio, on a mole basis, of the n-butyllithium to the ethyllithium is from 1:9 to 9:1; of the sec-butyllithium to the ethyllithium is from 17:83 to 95:5; of the isopropyllithium to the ethyllithium is from 1:9 to 9:1.

12. The composition of Claim 11, in which the liquid hydrocarbon solvent is selected from aliphatic and cycloaliphatic hydrocarbon solvents.

13. The composition of Claim 12, in which the complex is an NBEL complex, and the mole ratio of the n-butyllithium to the ethyllithium in said complex is equimolar.

14. The composition of Claim 12, in which the complex is an NBEL complex, and the mole ratio of the n-butyllitium to the ethyllithium in said complex is 35% to 65%.

15. The composition of Claim 12, in which the complex is an NBEL complex, and the mole ratio of the n-butyllithium to the ethyllithium in said complex is 25% to 75%.

16. The composition of Claim 12, in which the weight percentage of said complex is in the range of 20 to 93.

17. The composition of Claims 13, 14 or 15, in which the weight percentage of said complex is in the range of 12 to 25.

18. The composition of Claim 12, in which the complex is an SBEL complex, and the mole ratio of the sec-butyllithium to the ethyllithium in said complex is equimolar.

19. The composition of Claim 12, in which the complex is an SBEL complex, and the mole ratio of the sec-butyllithium to the ethyllithium in said complex 35% to 70%.

20. The composition of Claim 12, in which the complex is an SBEL complex, and the mole ratio of the sec-butyllithium to the ethyllithium in said complex is 25% to 75%.

21. The composition of Claims 18, 19 or 20, in which the weight percentage of said complex in in the range of 12 to 50.

22. The composition of Claim 12, in which the complex is a IPEL complex, and the mole ratio of the isopropyllithium to the ethyllithium in said complex is equimolar.

23. The composition of Claim 12, in which the complex is an IPEL complex, and the mole ratio of the isopropyllithium to the ethyllithium in said complex is 35% to 65%.

24. The composition of Claim 12, in which the complex is an IPEL complex, and the mole ratio of the isopropyllithium to the ethyllithium in said complex is 25% to 75%.

**Patentansprüche**

1. Verfahren zur Herstellung von Lösungen von Komplexen, ausgewählt aus
a) Butyllithium,
b) sek.-Butyllithium und
c) Isopropyllithium
zusammen mit Ethyllithium, wobei das Molverhältnis n-Butyllithium zu Ethyllithium 1:9 bis 9:1, dasjenige von sek.-Butyllithium zu Ethyllithium 17:83 bus 95:5 und dasjenige von Isopropyllithium zu Ethyllithium 1:9 bis 9:1 beträgt, in flüssigen Kohlenwasserstoffen, gekennzeichnet durch die Stufen des Herstellens einer gerührten Dispersion von feinverteiltem Lithiummetall, enthaltend eine kleine Menge Natriummetall in einem flüssigen Kohlenwasserstoff, ausgewählt aus aliphatischen und aromatischen Kohlenwasserstoffen, mit denen das Lithium im wesentlichen nicht reaktiv ist, des allmählichen und gleichzeitigen Zusetzens zur Lithiummetalldispersion unter Bedingungen des heftigen Rührens und in einer Inertgasatmosphäre von (a') n-Butylchlorid oder -bromid (b') sek.-Butylchlorid oder -bromid, (c') Isopropylchlorid oder -bromid, mit Ethylchlorid oder -bromid in vorbestimmten Mengenverhältnissen zueinander zur Schaffung der genannten Lösungen, welche die Komplexe (a), (b) oder (c) in den oben genannten Molverhältnissen enthalten, Fortsetzen der Reaktion bei einer Temperatur im Bereich von 20 bis 35°C unter Rühren, bis die Reaktion wenigstens im wesentlichen vollständig abgelaufen ist, und Filtrieren zu einer im wesentlichen klaren Lösung der genannten Komplexe (a), (b) oder (c) in dem Kohlenwasserstoff-Lösungsmittel.

2. Verfahren nach Anspruch 1, wobei das Molverhältnis (a), (b) oder (c) zu Ethyllithium in den Komplexen im wesentlichen äquimolar ist.

3. Verfahren nach Anspruch 1, wobei das Molverhältnis von (a), (b) oder (c) zu Ethyllithium 35 zu 65 beträgt.

4. Verfahren nach Anspruch 1, wobei das Molverhältnis von (a), (b) oder (c) zu Ethyllithium 25 zu 75 beträgt.

5. Verfahren nach Anspruch 1, wobei der Lithiumgehalt der Aufschlämmung einen Überschuß zur stöchiometrischen Menge bezüglich der Mengen von (a'), (b') oder (c'), je nach dem Fall beträgt.

6. Verfahren nach Anspruch 1, wobei der Gewichtsprozentsatz der Komplexe (a), (b) oder (c) im Kohlenwasserstoff-Lösungsmittel derart ist, daß eine verdünnte Lösung davon gebildet wird.

7. Verfahren nach Anspruch 6, wobei das Kohlenwasserstoff-Lösungsmittel ausgewählt ist an aliphatischen und/oder cycloaliphatischen Kohlenwasserstoff-Lösungsmitteln.

8. Verfahren nach Anspruch 7, wobei das Molverhältnis des n-Butyllithiums zum Ethyllithium in den Komplexen äquimolar ist.

9. Verfahren nach Anspruch 7, wobei das Molverhältnis des n-Butyllithiums zum Ethyllithium 25 zu 75 beträgt.

10. Verfahren nach Anspruch 1, wobei das Molverhältnisse von (a), (b) oder (c) zum Ethyllithium in den Komplexen ausgewählt sind aus (a) etwa äquimolar, (b) 35 zu 65 und (c) 25 zu 75 und wobei die Gewichtsprozentsätze der Komplexe in dem Kohlenwasserstoff-Lösungsmittel derart sind, daß eine verdünnte Lösung davon gebildet wird.

11. Zubereitung, bestehend aus einer klaren Lösung in einem flüssigen Kohlenwasserstoff-Lösungsmittel eines Komplexes, ausgewählt aus (a) n-Butyllithium, (b) sek.-Buthyllithium und (c) Isopropyllithium, zusammen mit Ethyllithium, wobei das Verhältnis, auf Molbasis, das n-Butyllithium zum Ethyllithium 1:9 bis 9:1, dasjenige des sek.-Butyllithiums zum Ethyllithium 17:83 bis 95:5 und dasjenige des Isopropyllithiums zum Ethyllithium 1:9 bis 9:1 beträgt.

12. Zubereitung nach Anspruch 11, wobei das flüssige Kohlenwasserstoff-Lösungsmittel ausgewählt ist aus aliphatischen und cycloaliphatischen Kohlenwasserstoff- Lösungsmitteln.

13. Zubereitung nach Anspruch 12, wobei der Komplex ein NBEL-Komplex ist und das Molverhältnis von n-Butyllithium zum Ethyllithium im Komplex äquimolar ist.

14. Zubereitung nach Anspruch 12, wobei der Komplex ein NBEL-Komplex ist und das Molverhältnis des n-Butyllithiums zum Ethyllithium im Komplex 35% bis 65% beträgt.

15. Zubereitung nach Anspruch 12, wobei der Komplex ein NBEL-Komplex ist und das Molverhältnis von n-Butyllithium zum Ethyllithium im Komplex 25% bis 75% beträgt.

16. Zubereitung nach Anspruch 12, wobei der Gewichtsprozentsatz des Komplex im Bereich von 20 bis 93 liegt.

17. Zubereitung nach den Ansprüchen 13, 14 oder 15, wobei der Gewichtsprozentsatz des Komplex im Bereich von 12 bis 25 liegt.

18. Zubereitung nach Anspruch 12, wobei der Komplex ein SBEL-Komplex ist und das Molverhältnis des sek.-Butyllithiums zum Ethyllithium im Komplex äquimolar ist.

19. Zubereitung nach Anspruch 12, wobei der Komplex ein SBEL-Komplex ist und das Molverhältnis des sek.-Butyllithiums zum Ethyllithium im Komplex 35% bis 70% beträgt.

20. Zubereitung nach Anspruch 12, wobei der Komplex ein SBEL-Komplex ist und das Molverhältnis des sek.-Butyllithiums zum Ethyllithiums im Komplex 25% bis 75% beträgt.

21. Zubereitung nach den Ansprüchen 18, 19 oder 20, wobei der Gewichtprozentsatz des Komplex im Bereich von 12 bis 50 liegt.

22. Zubereitung nach Anspruch 12, wobei der Komplex ein IPEL-Komplex ist und das Molverhältnis des Isopropyllithiums zum Ethyllithium im Komplex äquimolar ist.

23. Zubereitung nach Anspruch 12, wobei der Komplex ein IPEL-Komplex ist und das Molverhältnis des Isopropyllithiums zum Ethyllithium im Komplex 35% bis 65% beträgt.

24. Zubereitung nach Anspruch 12, wobei der Komplex ein IPEL-Komplex ist und das Molverhältnis des Isopropyllithiums zum Ethyllithium im Komplex 25% bis 75% beträgt.

**Revendications**

1. Une méthode de préparation de solutions dans un hydrocarbure liquide de complexes choisis parmi:
(a) le n-butyllithium, (b) le sec-butyllithium et (c) l'isopropyllithium, avec l'éthyllithium, les rapports en moles du n-butyllithium à l'éthyllithium variant de 1:9 à 9:1; du secbutyllithium à l'éthyllithium variant de 17:83 à 95:5; de l'isopropyllithium à l'éthyllithium variant de 1:9 à 9:1; qui comprend les étapes consistant à utiliser une dispersion agitée de lithium metal finement divisé contenant une petite proportion de sodium métal dans un hydrocarbure liquide choisi parmi les hydrocarbures aliphatiques et cycloaliphatiques avec lesquels le lithium métal est pratiquement non réactif, à ajouter simultanément, progressivement, à ladite dispersion de lithium métal, dans des conditions d'agitation vigoureuse et sous atmosphère de gaz inerte, (a') du chlorure ou bromure de n-butyle, (b') du chlorure ou bromure de sec-butyle, (c') du chlorure ou bromure d'isopropyle, avec du chlorure ou bromure d'éthyle en proportions relatives et prédéterminées pour fournir lesdites solutions contenant les complexes (a), (b) ou (c); dans les proportions molaires indiquées, et à continuer la réaction à une température dans la gamme de 20° à 35°, dans des conditions d'agitation, jusqu'à ce que laidte réaction soit sensiblement complète et à filtrer pour obtenir une solution sensiblement limpide desdits complexes (a), (b) ou (c) dans ledit solvant hydrocarbure.

2. La méthode de la revendication 1 dans laquelle le rapport molaire de (a), (b) ou (c) à l'éthyllithium dans lesdits complexes est sensiblement équimolaire.

3. La méthode de la revendication 1 dans laquelle le rapport molaire de (a), (b) ou (c) à l'éthyllithium est de 35 à 65.

4. La méthode de la revendication 1 dans laquelle le rapport molaire de (a), (b) ou (c) à l'éthyllithium est de 25 à 75.

5. La méthode de la revendication 1 dans laquelle la teneur en lithium de ladite dispersion est en excès stoéchiométrique par rapport aux teneurs en (a'), (b') ou (c') selon le cas.

6. La méthode de la revendication 1 dans laquelle le pourcentage en poids desdits complexes de (a), (b) ou (c) dans ledit solvant hydrocarbure est tel que l'on obtient une solution diluée desdits complexes.

7. La méthode de la revendication 6 dans laquelle ledit solvant hydrocarbure est choisi parmi les hydrocarbures aliphatiques et/ou cycloaliphatiques.

8. La méthode de la revendication 7 dans laquelle le rapport molaire du n-butyllithium à l'éthyllithium dans lesdits complexes est équimolaire.

9. La méthode de la revendication 7 dans laquelle le rapport molaire du n-butyllithium à l'éthyllithium est de 25 à 75.

10. La méthode de la revendication 1 dans laquelle les rapports molaires de (a), (b) ou (c) à l'éthyllithium dans lesdits complexes est, pour (a) sensiblement équimolaire, pour (b) de 35 à 65 et pour (c) de 25 à 75 et dans laquelle les pourcentages en poids desdits complexes dans le solvant hydrocarbure sont tels qu'on obtient une solution diluée.

11. Une composition comprenant une solution limpide dans un solvant hydrocarbure liquide d'un complexe choisi parmi les complexes (a) du n-butyllithium, (b) du sec-butyllithium et (c) de l'isopropyllithium avec l'éthyllithium, dans laquelle le rapport molaire du n-butyllithium à l'éthyllithium varie de 1:9 à 9:1; du sec-butyllithium à l'éthyllithium varie de 17:83 à 95:5; de l'isopropyllithium à l'éthyllithium varie de 1:9 à 9:1.

12. La composition de la revendication 11 dans laquelle ledit solvant hydrocarbure liquide est choisi parmi les hydrocarbures aliphatiques et cycloaliphatiques.

13. La composition de la revendication 12 dans laquelle le complexe est un complexe NBEL, le rapport molaire du n-butyllithium à l'éthyllithium dans ledit complexe étant équimolaire.

14. La composition de la revendication 12 dans laquelle le complexe est un complexe NBEL, le rapport molaire du n-butyllithium à l'éthyllithium dans ledit complexe étant de 35% à 65%.

15. La composition de la revendication 12 dans laquelle le complexe est un complexe NBEL, le rapport molaire du n-butyllithium à l'éthyllithium dans ledit complexe étant de 25% à 75%.

16. La composition de la revendication 12 dans laquelle le pourcentage en poids dudit complexe est dans la gamme de 20 à 93.

17. La composition de la revendication 13, 14 ou 15 dans laquelle le pourcentage en poids dudit complexe est dans la gamme de 12 à 25.

18. La composition de la revendication 12 dans laquelle le complexe est un complexe SBEL et le rapport molaire du sec-butyl-lithium à l'éthyllithium dans ledit complexe est équimolaire.

19. La composition de la revendication 12 dans laquelle le complexe est un complexe SBEL et le rapport molaire du sec-butyllithium à l'éthyllithium dans ledit complexe est de 35% à 70%.

20. La composition de la revendication 12 dans laquelle le complexe est un complexe SBEL et le rapport molaire du sec-butyllithium à l'éthyllithium dans ledit complexe est de 25% à 75%.

21. La composition de la revendication 18, 19 ou 20 dans laquelle le pourcentage en poids dudit complexe est dans la gamme de 12 à 50.

22. La composition de la revendication 12 dans laquelle le complexe est un complexe IPEL et le rapport molaire de l'isopropyllithium à l'éthyllithium dans ledit complexe est équimolaire.

23. La composition de la revendication 12 dans laquelle le complexe est un complexe IPEL et le rapport molaire de l'isopropyllithium à l'éthyllithium dans ledit complexe est de 35% à 65%.

24. La composition de la revendication 12 dans laquelle le complexe est un complexe IPEL et le rapport molaire de l'isopropyllithium à l'éthyllithium dans ledit complexe est de 25% à 75%.